# EUROPEAN PATENT APPLICATION

(11) **EP 3 888 621 A1**
(43) Date of publication of application: **06.10.2021**
(21) Application number: 20167828.1
(22) Date of filing: 02.04.2020
(51) Int. Cl.: A61K 8/02, A61C 19/06, A61K 8/25, A61K 8/73, A61K 8/81, A61Q 11/00, A61K 8/24

(54) **ORAL CARE DEVICE**

(71) Applicant: Unilever Global IP Ltd, Wirral, CH62 4ZD (GB)
(72) Inventor: GROVES, Brian Joseph, Merseyside, CH63 3JW (GB); LIMER, Adam John, Merseyside, CH63 3JW (GB)
(74) Representative: Tansley, Sally Elizabeth

(57) **Abstract**

A system for whitening the teeth, the system comprising a delivery device and an activator composition, the delivery device comprising;
i) a strip of an orally acceptable flexible material, having a strip surface capable of being applied to a tooth surface;
ii) a phosphate source;
iii) an adhesive comprising a hydroxy cellulose and polyethylene glycol.

## Description

### Field of the Invention

The invention relates to an oral care product for the remineralisation and whitening of teeth.

### Background of the Invention

Teeth are important both functionally and aesthetically. There is a need for strong healthy teeth that appear white and glossy.

WO2016/192923 discloses a delivery device for delivering an enamel regeneration system to the surfaces of teeth. The device is a strip having the enamel regeneration system deposited upon the strip surface thereof, and/or impregnated into its structure. Similarly whitening and enamel regeneration systems comprising a strip are disclosed in WO2016/192924 and WO2016/19295.

However there remains the need for a simple and effective way to adhere strips to the teeth in which the sensory feel of the strip is acceptable.

### Summary of the Invention

Accordingly, the present invention relates to a system for whitening the teeth the system comprising a delivery device and an activator composition, the delivery device comprising:
i) a strip of an orally acceptable flexible material, having a strip surface capable of being applied to a tooth surface;
ii) a phosphate source;
iii) an adhesive comprising a hydroxy cellulose and polyethylene glycol.

### Detailed Description of the Invention

The delivery device of the invention comprises a strip of an orally acceptable flexible material. The surface of the strip is capable of being applied to a tooth surface.

The delivery device comprises an adhesive layer. This adhesive layer comprises hydroxy cellulose and polyethylene glycol. Preferably the hydroxy cellulose of the adhesive layer is a hydroxy alkyl cellulose, more preferably hydroxy ethyl cellulose. Preferably the polyethylene glycol has a molecular weight (Mn) from 500 to 4 000, more preferably from 1000 to 2000.

Preferably the weight ratio of hydroxy cellulose to polyethylene glycol is from 1:2 to 1:30, more preferably form 1:3 to 1:20.

Preferably the device comprises a phosphate source. The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 1 to 12%, and most preferably, from 2 to 9% by weight of the composition used in the third layer, based on total weight of the composition of the third layer and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

Preferably the device has an elongate shape of a length sufficient that when placed against the front surface of a user's teeth it extends across a plurality of teeth, and of sufficient width that it extends at least from the gumline of the teeth to the crowns of the teeth. The elongate shape is such that it minimises the need for subsequent applications and time to cover all the user's teeth.

In a further embodiment, the strip is such that it can be sufficient that when placed against the front surface of a user's teeth it extends across a plurality of teeth, and of sufficient width that it extends at least from the front gumline of the teeth to the crowns of the teeth and to the gumline behind the user's teeth leading to total coverage of the teeth above the gumline.

Preferably the application device is rectangular in shape.

Preferably the strip/device has the enamel regeneration system deposited upon the strip surface thereof as a layer or more preferably multiple layers.

In a preferred embodiment, the device is a strip comprising a plurality of layers.

It is preferable if the thickness of the first layer is less than about 1 mm thick, preferably less than about 0.05 mm thick, and more preferably from about 0.001 to about 0.03 mm thick, second layer is less than about 1 mm thick, preferably less than about 0.5 mm thick, and more preferably from about 0.001 to about 0.3 mm thick and third layer (if present) is less than about 1 mm thick, preferably less than about 0.5 mm thick, and more preferably from about 0.001 to about 0.3 mm thick.

Preferably the device comprises at least two layers; one layer comprises a non-dissolving backing film, a second layer comprises a component of the enamel regeneration system. More preferably the strip comprises third protective layer it is particularly preferred if the third protective layer comprises a water-soluble polymer.

It is preferable if the non-dissolving backing layer comprises materials such as polymers, natural and synthetic wovens, non-wovens, foil, paper, rubber, and combinations thereof. The strip of material may be a single layer of material or a laminate of more than one layer. Generally, the strip of material is substantially water impermeable. The material may be any type of polymer that is compatible with tooth whitening actives and is sufficiently flexible to be shaped and applied to the tooth surface. The material may comprise a single polymer or a mixtures of polymers. Suitable polymers include, but are not limited to, polyethylene, polypropylene, ethylvinylacetate, ethylvinyl alcohol, polyesters, polyamides, fluoroplastics and combinations thereof. Preferably, the material is polyethylene, more preferably polyethylene terephthalate.

The strip of material is generally less than about 1 mm thick, preferably less than about 0.05 mm thick, and more preferably from about 0.001 to about 0.03 mm thick.

The advantage of having a water-soluble layer and a non-water soluble layer is that in the mouth the water-soluble layer dissolves in the saliva and the ingredients enclosed therein (the phosphate) can react with the ingredients in the second layer (silicate) to aid the in-situ regeneration of enamel on the teeth. Thus, the system provides an enamel regeneration system that can be used with the minimum of water, can be easily stored and is stable on storage.

Preferably the system according to the invention comprises an application device and ad activating system. The activator system preferably comprises an anhydrous base formulation. The activator serum preferably comprises a phosphate source. The phosphate source that may be used in this invention is limited only to the extent that the same may be used in a composition suitable for use in an oral cavity. Illustrative examples of the types of phosphate source suitable for use in this invention include monosodium phosphate, sodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate, mixtures thereof or the like. The phosphate source is preferably one which is water soluble.

Typically, the phosphate source makes up from 0.5 to 15%, and preferably, from 1 to 12%, and most preferably, from 2 to 9% by weight of the composition used in the third layer, based on total weight of the composition of the third layer and including all ranges subsumed therein. In a preferred embodiment, the phosphate source used is one which results in an oral care composition having a pH from 5.5 to 8, preferably from 6 to 7.5, and most preferably, about neutral. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The oral care composition described herein may comprise ingredients which are common in the art, such as:
▪ antimicrobial agents, e.g. Triclosan, chlorhexidine, copper-, zinc- and stannous salts such as zinc citrate, zinc sulphate, zinc glycinate, sodium zinc citrate and stannous pyrophosphate, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds such as 2,2' methylenebis-(4-chloro-6-bromophenol);
▪ anti-inflammatory agents such as ibuprofen, flurbiprofen, aspirin, indomethacin, etc.;
▪ anti-caries agents such as sodium trimetaphosphate and casein;
▪ plaque buffers such as urea, calcium lactate, calcium glycerophosphate and polyacrylates;
▪ vitamins such as Vitamins A, C and E;
▪ plant extracts;
▪ desensitizing agents, e.g. potassium citrate, potassium chloride, potassium tartrate, potassium bicarbonate, potassium oxalate, and potassium nitrate;
▪ anti-calculus agents, e.g. alkali-metal pyrophosphates, hypophosphite-containing polymers, organic phosphonates and phosphocitrates, etc;
▪ biomolecules, e.g. bacteriocins, antibodies, enzymes, etc ;
▪ flavours, e.g., peppermint and spearmint oils;
▪ proteinaceous materials such as collagen;
▪ preservatives;
▪ opacifying agents;
▪ colouring agents like FD&C blue, yellow and/or red dyes/colorants;
▪ pH-adjusting agents;
▪ sweetening agents;
▪ surfactants, such as anionic, cationic and zwitterionic or amphoteric surfactants (e.g., sodium lauryl sulfate, sodium dodecylbenzene sulfonate);
▪ particulate abrasive materials such as abrasive silicas, aluminas, calcium carbonates, zirconium silicate, polymethylmethacrylate, dicalciumphosphates, calcium pyrophosphates, hydroxyapatites, trimetaphosphates, insoluble hexametaphosphates as well as agglomerated particulate abrasive materials;
▪ fluoride sources like sodium fluoride, stannous fluoride, sodium monofluorophosphate, zinc ammonium fluoride, tin ammonium fluoride, calcium fluoride, cobalt ammonium fluoride or mixtures thereof;
▪ polymeric compounds which can enhance the delivery of active ingredients such as antimicrobial agents can also be included. Examples of such polymers are copolymers of polyvinylmethylether with maleic anhydride and other similar delivery enhancing polymers, e.g., those described in DE-A03,942,643; buffers and salts to buffer the pH and ionic strength of the oral care compositions; and
▪ other optional ingredients that may be included are, e.g., bleaching agents such as peroxy compound, e.g., potassium peroxydiphosphate, effervescing systems such as sodium bicarbonate/citric acid systems, colour change systems, and the like.

Such ingredients common in the art typically and collectively make-up less than 20% by weight of the oral care composition, and preferably, from 0.0 to 15% by weight, and most preferably, from about 0.01 to about 12% by weight of the oral care composition, including all ranges subsumed therein.

Suitable carrier humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. The carrier humectants should, in any case, be substantially free of water, and preferably, anhydrous. The same, for example, can be used in solid form, whereby glycerin is the preferred carrier humectant. Such carriers are particularly suitable in compositions used in the second layer.

The carrier humectant is used to take the balance of the compositions up to 100%, and the same may be present in the range of from 10 to 90% by weight of the oral care composition. Preferably, the carrier humectant makes up from 25 to 80%, and most preferably, from 45 to 70% by weight of the oral care composition, based on total weight of the oral care composition and including all ranges subsumed therein.

The composition used in the layers of the invention are prepared by conventional methods of making oral care formulations. Such methods include mixing the ingredients under moderate shear and atmospheric pressure.

Preferably the system according to the invention, more preferably the activating composition comprises an insoluble and/or slightly soluble calcium source, soluble as used herein, refers to the solubility of the calcium source in water. Soluble means a source that dissolves in water to give a solution with a concentration of at least 0.1 moles per litre at room temperature. Insoluble means a source that dissolves in water to give a solution with a concentration of less than 0.001 moles per litre at room temperature. Slightly soluble, therefore, is defined to mean a source that dissolves in water to give a solution with a concentration of greater than 0.001 moles per litre at room temperature and less than 0.1 moles per litre at room temperature. Substantially free of, as used herein, means less than 1.5%, and preferably, less than 1.0%, and most preferably, from 0.0 to 0.75% by weight, based on total weight of the oral care composition, including all ranges subsumed therein. The calcium source suitable for use in this invention is limited only to the extent that the same may be used in an oral cavity. In a preferred embodiment, the calcium source employed is insoluble or slightly soluble in water, but most preferably, insoluble in water.

Illustrative examples of the types of calcium source that may be used in this invention include, for example, calcium phosphate (i.e., added), calcium gluconate, calcium oxide, calcium lactate, calcium carbonate, calcium hydroxide, calcium sulfate, calcium carboxymethyl cellulose, calcium alginate, calcium salts of citric acid, calcium silicate, mixtures thereof or the like. In a preferred embodiment, the calcium source is calcium silicate. In a more preferred embodiment, the calcium silicate used is (CaSᵢO₃) whereby the same is made commercially available under the name Microcal ET by Ineos Silicas, Ltd.

In yet another preferred embodiment, the calcium source is insoluble calcium silicate, present as the composite material calcium oxide-silica (CaO-SiO₂) as described in commonly-owned application Publication No. 2008/015117.

When a calcium silicate composite material is employed, the ratio of calcium to silicon (Ca:Si) may be from 1:10 to 3:1. The Ca:Si ratio is preferably from 1:5 to 2:1, and more preferably, from 1:3 to 2:1, and most preferably, from about 1:2 to 2:1. The calcium silicate may comprise mono-calcium silicate, bi-calcium silicate, or tri-calcium silicate whereby ratios of calcium to silicon (Ca:Si) should be understood to be atom ratios.

The calcium source employed in this invention may be in a crystalline or amorphous state, and preferably, the same is in an amorphous state. In an often-preferred embodiment, the calcium source is in a mesoporous state, i.e. the source is a material having pores with diameters from 1 nm to 50 microns. Mesoporous calcium silicate (MCS) is often preferred.

The MCS which may be used in this invention can be made by combining a calcium salt, a silica precursor like silicate and a structure-directing agent to yield a solid suitable for calcinating. A more detailed description of the process that may be conducted to make the MCS suitable for use in this invention is described in the aforementioned commonly-owned application, Publication No. WO 2008/015117.

The amount of calcium source in the composition is typically from 0.1 to 50%, and preferably, from 1 to 30%, and most preferably, from 5 to 20% by weight of the oral care activating composition based on total weight of the oral care composition and including all ranges subsumed therein.

A preferred form of calcium silicate is calcium silicate coated TiO₂. Examples of preferred forms of calcium silicate coated TO02 are disclosed in WO2012/031786 and WO2012/031785.

Preferably the activating system comprises a phosphate source as described above. More preferably the same phosphate is present in the activating system as in the device.

### Mode of Use

The invention provides a method of whitening and remineralising teeth. The activator serum is applied to the strips of the invention. The activated strips as described above are applied to the surface of the teeth in sustained contact.

When an activator composition is used the activator composition is placed on the strip prior to placing the strip on the teeth.

In the context of the present invention sustained contact means the product is left on the teeth for 1 to 60 minutes, preferably, about 5 to 45 minutes, more preferably 10 to 30 minutes before being removed.

Preferably the application of the oral care product of the invention is carried out once daily for a period of several consecutive days, in addition to a regular regime of tooth brushing (preferably at least twice daily).

Typically, use (for a period of about two weeks to one month) of the device with the oral care composition of the present invention will result in a new hydroxyapatite layer on teeth that is from 0.5 to 20 microns, and preferably, from 0.75 to 5 microns, including all ranges subsumed therein.

### Detailed Description of Non-limiting Embodiments

The invention will now be illustrated by the following non-limiting Examples:

### Examples

### Example 1

Polymers were dispersed in water using an overhead bench top stirrer. A 3cm x 6.5cm slip of woven material was immersed into the polymer solution. The fabric slips were then placed on a glass tile and allowed to dry. Once dry the slips were examined to assess whether or not they had stuck to the glass.

| **No** | **Polymer** | **Adhesion to Glass** |
|---|---|---|
| 1 | Carrageenan | Good adhesion |
| 2 | Poly(ethylene)oxide | Poor adhesion |
| 3 | Hydroxy Propyl Methyl Cellulose | Poor adhesion |
| 4 | Polyvinylpyrrolidone | Poor adhesion |
| 5 | Xanthan Gum | Good adhesion |
| 6 | Sodium Carboxy Methyl Cellulose | Good adhesion |
| 7 | Hydroxyethylcellulose | Good adhesion |
| 8 | Guar Gum | Good adhesion |
| 9 | Locust Bean Gum | Good adhesion |
| 10 | Locust Bean Gum + Guar Gum | Good adhesion |
| 11 | Polystyrene sulfonate, sodium salt | Poor adhesion |
| 12 | Pluronic F127 - Poly(propylene glycol)-b-poly(ethylene glycol)-b-Poly(propylene glycol) | Poor adhesion |
| 13 | PolyCarbophil - high molecular weight crosslinked poly(acrylic acid) | Good adhesion |

Due to poor initial adhesion poly(ethylene)oxide, hydroxypropylmethyl cellulose, polyvinylpyrrolidone, polystyrene sulfonate sodium salt and Pluronic F127 were not considered for further testing.

It is desirable that the fabric is adhered to the glass as this mimics being adhered to a carrier film in packaging. It is also necessary that when re-wet the sample sticks to a surface again, so the material can be applied in the oral environment. To simulate this the removed fabric slips were dosed with 0.5g of water and placed on a clean glass tile and allowed to fully dry. The slips were then re-examined for adhesion to the glass tile.

| **No** | **Polymer** | **Adhesion to Glass** |
|---|---|---|
| 1 | Carrageenan | Good adhesion |
| 5 | Xanthan Gum | Good adhesion |
| 6 | Sodium Carboxy Methyl Cellulose | Good adhesion |
| 7 | Hydroxyethylcellulose | Good adhesion |
| 8 | Guar Gum | Poor adhesion |
| 9 | Locust Bean Gum | Poor adhesion |
| 10 | Locust Bean Gum + Guar Gum | Poor adhesion |
| 13 | PolyCarbophil - high molecular weight crosslinked poly(acrylic acid) | Good adhesion |

Due to poor repeated guar gum and locust bean gum were not considered for further testing.

When dried polymers may reduce the flexibility of the fabric and make it more difficult to apply in the oral environment. To enhance the flexibility of the fabric slip a plasticizer should be incorporated into the polymer solution. To test the effect of this 1wt% glycerol was added to polymer solutions. As previously the slips were then coated in the polymer solution, placed on the glass tile, and allowed to dry fully. All samples were examined for adhesion to the glass surface and then removed to confirm whether the adhesive remains on the fabric. It is desirable for the polymer to remain on the fabric slip and not be deposited on the glass surface.

| **No** | **Polymer** | **Adhesion to Glass** |
|---|---|---|
| 1 | Carrageenan | Good adhesion, polymer on glass and material. |
| 5 | Xanthan Gum | Good adhesion. |
| 6 | Sodium Carboxy Methyl Cellulose | Good adhesion. |
| 7 | Hydroxyethylcellulose | Good adhesion. |
| 13 | PolyCarbophil - high molecular weight crosslinked poly(acrylic acid) | Tacky, polymer on glass and material. |

The results indicate that xanthan gum, sodium carboxy methyl cellulose and hydroxyethyl cellulose are particularly desirable to use as an adhesive.

### Example 2

A simulated oral fluid was prepared by adding 1.9L of water to a glass beaker. The following materials were added one by one with continuous stirring, allowing sufficient time for each chemical to fully dissolve before adding the next. Sodium chloride 16.07g, sodium hydrogen carbonate 0.7g, potassium chloride 0.448g, potassium hydrogen phosphate 2.56g, magnesium chloride hexahydrate 0.622g, 1M hydrochloric acid 40ml, calcium chloride 0.1998g and sodium sulfate 0.1434g. The pH was adjusted to 7.0 using saturated TRIS buffer and the total volume made up to 2L using a volumetric flask.

Human extracted incisors and premolars, obtained for research purposes and obtained with informed consent in accordance with the Human Tissue Act were mounted in plastic cuvettes using the following method. The cuvettes were cut to approximately 15mm height. A commercial fixative (Simplex Rapid) was mixed 2 parts powder to 1 part liquid as described in the manufacturer's instructions. The cuvettes were then completely filled with the resultant solution and left for approximately 10 minutes to allow the fixative to partly set. At this time, one tooth root was completely immersed into the fixative, ensuring that the labial side of the tooth was positioned close to the front of the cuvette. Complete setting of the fixative was achieved after 20 minutes. Any exposed dentine is then sealed with clear nail varnish. The teeth are stored in water to prevent dehydration.

A formulation was prepared by combining the following:

**Formulation 1:**

| **Ingredient** | **Formulation 1 %w/w** |
|---|---|
| Glycerol | 31.245 |
| PEG 400 | 31.245 |
| Calcium Silicate | 11 |
| Calcium silicate coated TiO2 | 20 |
| Sodium monofluorophosphate | 1.11 |
| Calcium Phosphate | 2 |
| Sodium Saccharine | 0.1 |
| Flavor | 0.3 |
| Anhydrous Mono Sodium Phosphate | 1.50 |
| Anhydrous Tri Sodium Phosphate | 1.50 |
| Total | 100 |

The glycerol and PEG 400 were added to the Esco-Labor 1L mixing vessel and stirring at room temperature the powders are added slowly and mixed to remove any lumps.

A strip was produced by preparing two formulations:

**Formulation 2:**

| **Ingredient** | **%w/w** |
|---|---|
| Purified Water | 82.248% |
| Titanium Dioxide | 0.500% |
| Glycerine | 2.000% |
| PEG-1500 NF (Polyethylene Glycol 1500) | 12.000% |
| White Beeswak, NF | 0.500% |
| PEG-40 Hydrogenated Castor Oil | 0.250% |
| Benzyl Alcohol EP/USP/NF | 0.001% |
| Phenoxyethanol | 0.001% |
| Hydroxyethyl Cellulose | 2.500% |
| Total | 100% |

Water was added to a mixing vessel followed by PEG-40 Hydrogenated Castor Oil, glycerine, PEG, beeswax, Benzyl Alcohol, Phenoxyethanol, titanium dioxide and mixed to disperse. Subsequently hydroxyethyl cellulose was slowly added to the vessel and mixed to disperse.

**Formulation 3:**

| **Ingredient** | **%w/w** |
|---|---|
| Purified Water | 58.100% |
| Glycerine | 16.000% |
| Hydroxyethyl Cellulose | 1.000% |
| Silica | 17.830% |
| Polyvinylpyrrolidone | 2.000% |
| Trisodium Phosphate | 1.750% |
| Monosodium Phosphate | 1.750% |
| Aroma | 0.250% |
| Benzyl Alcohol | 0.300% |
| Phenoxyethanol | 0.300% |
| Ethylhexylglycerin | 0.300% |
| Sodium Fluoride | 0.220% |
| PEG-40 Hydrogenated Castor Oil | 0.100% |
| Sodium Saccharin | 0.100% |
| Total | 100% |

Water was added to a mixing vessel followed by PEG-40 Hydrogenated Castor Oil, glycerine, ethylhexyl glycerine, phenoxyethanol, Benzyl Alcohol, flavour, monosodium phosphate, trisodium phosphate, sodium fluoride, sodium saccharine, silica and mixed to disperse. Subsequently hydroxyethyl cellulose and polyvinylpyrrolidone was slowly added to the vessel and mixed to disperse.

A film of formulation 2 was applied to a PET film and dried in an oven to a water content of 28-30wt%. On this film was placed a layer of non-woven Rayon fabric and a film of formulation 3 applied. Again, the sample was dried in an oven to a water content of 28-30wt%. The resulting structure has four layers, a PET film, a layer of formulation 3, a layer or rayon and a layer of formulation 4. The resulting structure can be pealed from the PET layer to give a three-layer structure.

The product was tested as follows:
A 3:1 water:tooth-paste slurry was prepared using a Signal toothpaste marketed as Signal Anti-Caries. Each tooth was brushed by hand using a toothbrush for 5 seconds and soaked in slurry for 115 seconds. After the teeth were rinsed in 40 ml water and mixed gently for 1 minute. Two pieces of the four-layer structure were cut to a 10 x 3.5cm piece. After brushing the teeth with toothpaste and immediately before use, 0.5g of Formulation 1 was applied to the exposed silica layer of the fabric sample using a dropping pipette and spread evenly with a spatula. The sample was pealed from the PET carrier and wrapped around 5 human teeth for 15 minutes at 37°C. After the samples were removed from the fabric, rinsed in 40 ml water and mixed gently for 1 minute. Samples were then incubated in simulated oral fluid for 5 hours. The application process was repeated 5 times in total. Colour was measured via chromameter at baseline and after slurry application and incubation. L*a*b* colour parameters were converted to WIO whiteness indices to allow comparison between samples. Colour change is expressed as ΔWIO = WIO(slurry application)-WIO(baseline).

| | **1 Application** | **5 Applications** |
|---|---|---|
| Formulation 1,2 & 3 | 9.38 ± 1.18 | 18.66 ± 3.43 |

The example demonstrates that product can effectively whiten teeth.

## Claims

1. A system for whitening the teeth, the system comprising a delivery device and an activator composition, the delivery device comprising;
i) a strip of an orally acceptable flexible material, having a strip surface capable of being applied to a tooth surface;
ii) a phosphate source;
iii) an adhesive comprising a hydroxy cellulose and polyethylene glycol.

2. A system according to claim 1, the device comprising a plurality of layers.

3. A system according to claim 2 or claim 3, in which one layer of the device comprises a non-dissolving backing film.

4. A system according to claim 3 in which the non-dissolving backing film comprises polyethylene terephthalate.

5. A system according to any preceding claim in which the hydroxy cellulose of the adhesive layer is hydroxy ethyl cellulose.

6. A system according to any preceding claim in which the polyethylene glycol has a molecular weight (Mn) from 500 to 4000.

7. A system according to any preceding claim in which the phosphate source comprises trisodium phosphate, sodium dihydrogen phosphate or mixtures thereof.

8. A system according to any preceding claim in which the activator composition comprises a water insoluble and/or slightly soluble calcium source.

9. A system according to claim 8 in which the calcium source of the enamel regeneration system is calcium silicate.

10. A system according to any preceding claim in which the activator composition comprises a phosphate source.

11. A system according to any preceding claim in which the activator composition comprises a tooth whitening substance is calcium silicate coated TiO2.

12. A system according to any preceding claim in which the device has an elongate shape of a length sufficient that when placed against the front surface of a user's teeth it extends across a plurality of teeth, and of sufficient width that it extends at least from the gumline of the teeth to the crowns of the teeth.

13. A system according to any preceding claim in which the device is substantially rectangular.
